# EUROPEAN PATENT APPLICATION

(11) **EP 2 228 367 A1**
(43) Date of publication of application: **15.09.2010**
(21) Application number: 09382031.4
(22) Date of filing: 13.03.2009
(51) Int. Cl.: C07D 213/80, A61K 31/455, A61P 37/00

(54) **Addition salts of amines containing hydroxyl and/or carboxylic groups with amino nicotinic acid derivatives as DHODH inhibitors**

(71) Applicant: Almirall, S.A., 08022 Barcelona (ES)
(72) Inventor: Perez Garcia, Juan Bautista, 08980, SANT FELIU DE LLOBREGAT (Barcelon (ES); Carrera Carrera, Francesc, 08980, SANT FELIU DE LLOBREGAT (Barcelon (ES); Garcia Martin, Digna Jose, 08980, SANT FELIU DE LLOBREGAT (Barcelon (ES); Boix Bernardini, Maria Carmen, 08980, SANT FELIU DE LLOBREGAT (Barcelon (ES)
(74) Representative: Elzaburu Marquez, Alberto

(57) **Abstract**

The present invention is directed to novel water-soluble pharmaceutically acceptable, crystalline addition salts of (i) an amine containing one or more hydroxyl and/or carboxylic groups with (ii) amino nicotinic acid derivatives of formula (I) 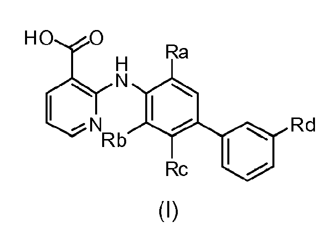 wherein R^{a}, R^{b}, R^{c} and R^{d} independently represent groups selected from hydrogen atoms, halogen atoms, C₁₋₄ alkyl groups which may be optionally substituted by 1, 2 or 3 substituents selected from halogen atoms and hydroxy groups, and C₁₋₄ alkoxy groups which may be optionally substituted by 1, 2 or 3 substituents selected from halogen atoms and hydroxy groups, and pharmaceutically acceptable solvates thereof.

## Description

### FIELD OF THE INVENTION

The present invention is directed to novel water-soluble pharmaceutically acceptable, crystalline addition salts of (i) amines containing one or more hydroxyl and/or carboxylic groups with (ii) amino nicotinic acid derivatives and solvates thereof. The invention is also directed to pharmaceutical compositions comprising the salts, methods of using them to treat, prevent or suppress diseases and disorders susceptible to be ameliorated by inhibition of dihydroorotate dehydrogenase, and processes and intermediates useful for preparing such salts.

### BACKGROUND OF THE INVENTION

Dihydroorotate dehydrogenase (DHODH) inhibitors are compounds useful in the treatment, prevention or suppression of diseases and disorders known to be susceptible to improvement by inhibition of dihydroorotate dehydrogenase, such as autoimmune diseases, immune and inflammatory diseases, destructive bone disorders, malignant neoplastic diseases, angiogenic-related disorders, viral diseases, and infectious diseases.

In view of the physiological effects mediated by inhibition of dihydroorotate dehydrogenase, several DHODH inhibitors have been recently disclosed for the treatment or prevention of the diseases or disorders indicated above. See for example, WO2006/044741; WO2006/022442; WO2006/001961, WO2004/056747, WO2004/056746, WO2003/006425, WO2002/080897 and WO99/45926.

One of the most challenging tasks for formulators in the pharmaceutical industry is incorporating poorly water-soluble drugs into effective pharmaceutical compositions intended for parenteral, e.g. intravenous, or oral administration.

Additionally, the aqueous solubility of poorly water-soluble drugs is an important factor affecting their bioavailability. Improving the solubility of these poorly water-soluble drugs may be achieved using a number of different systems (emulsions, microemulsions, self-emulsifying or micronization). However, all of these systems may need the presence of surfactants to solubilize or emulsify the drugs.

The solubility of poorly water-soluble drugs might also be improved by preparing their addition salts. However, in some cases unstable salts are formed due to hygroscopicity (the process by which a substance attracts moisture from the atmosphere by through either absorption or adsorption) or deliquescence (the process by which a substance absorbs moisture from the atmosphere until it dissolves in the absorbed water and forms a solution)

WO2008/077639 discloses new amino nicotinic acid and isonicotinic acid derivatives as potent DHODH inhibitors. Although these compounds have shown adequate pharmacological activity, they are poorly water soluble.

Accordingly, there is a need for water soluble DHODH inhibitors, which are also soluble in the gastrointestinal pH range, and in a physically and chemically stable, non-deliquescent form with acceptable levels of hygroscopicity and relative high melting point. This would allow the material to be further manipulated, e.g. by micronization without significant decomposition, loss of crystallinity or exhibiting any change in polymorphism to prepare pharmaceutical compositions and formulations.

### SUMMARY OF THE INVENTION

It has now been found that water-soluble addition salts of amines containing one or more hydroxyl and/or carboxylic groups with amino nicotinic acid derivatives are water-soluble and can be obtained in a crystalline form which is neither hygroscopic nor deliquescent and which has a relatively high melting point.

Thus, the present invention provides a pharmaceutically acceptable, crystalline addition salt of (i) an amine containing one or more hydroxyl and/or carboxylic groups with (ii) an amino nicotinic acid derivative of formula (I) 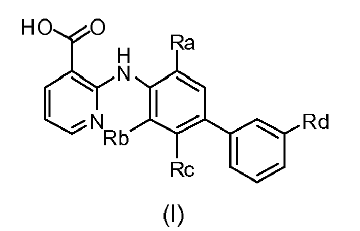 wherein
R^{a}, R^{b}, R^{c} and R^{d} independently represent groups selected from hydrogen atoms, halogen atoms, C₁₋₄ alkyl groups which may be optionally substituted by 1, 2 or 3 substituents selected from halogen atoms and hydroxy groups, and C₁₋₄ alkoxy groups which may be optionally substituted by 1, 2 or 3 substituents selected from halogen atoms and hydroxy groups,
and pharmaceutically acceptable solvates thereof.

The invention also provides a pharmaceutical composition comprising a salt of the invention and a pharmaceutically-acceptable carrier. The invention further provides combinations comprising a salt of the invention and one or more other therapeutic agents and pharmaceutical compositions comprising such combinations.

The invention also provides a method of treatment of a pathological condition or disease susceptible to amelioration by inhibition of dihydroorotate dehydrogenase, in particular wherein the pathological condition or disease is selected from rheumatoid arthritis, psoriatic arthritis, ankylosing spondilytis, multiple sclerosis, Wegener's granulomatosis, systemic lupus erythematosus, psoriasis and sarcoidosis, comprising administering a therapeutically effective amount of a salt of the invention. The invention further provides a method of treatment comprising administering a therapeutically effective amount of a combination of a salt of the invention together with one or more other therapeutic agents or administering a therapeutically effective amount of a pharmaceutical composition comprising such combination.

The invention further provides synthetic processes and intermediates described herein, which are useful for preparing salts of the invention.

The invention also provides a salt of the invention as described herein, a combination of a salt of the invention together with one or more other therapeutic agents or a pharmaceutical composition comprising such combination for use in the treatment of a pathological condition or disease susceptible to amelioration by inhibition of dihydroorotate dehydrogenase, in particular wherein the pathological condition or disease is selected from rheumatoid arthritis, psoriatic arthritis, ankylosing spondilytis, multiple sclerosis, Wegener's granulomatosis, systemic lupus erythematosus, psoriasis and sarcoidosis. The invention also provides the use of the salt of the invention, a combination of a salt of the invention together with one or more other therapeutic agents or a pharmaceutical composition comprising such combination for the manufacture of a formulation or medicament for treating these diseases.

### BRIEF DESCRIPTION OF FIGURES

Figure 1 illustrates the DSC thermogram of 2-[(3,5-difluoro-3'-methoxy-1,1'-biphenyl-4-yl)amino]nicotinic acid, meglumine salt.
Figure 2 illustrates the DVS pattern of 2-[(3,5-difluoro-3'-methoxy-1,1'-biphenyl-4-yl)amino]nicotinic acid, meglumine salt.
Figure 3 illustrates the IR spectra of 2-[(3,5-difluoro-3'-methoxy-1,1'-biphenyl-4-yl)amino]nicotinic acid, meglumine salt.
Figure 4 illustrates the DSC thermogram of 2-[(3,5-difluoro-3'-methoxy-1,1'-biphenyl-4-yl)amino]nicotinic acid, tromethamine salt.
Figure 5 illustrates the DVS pattern of 2-[(3,5-difluoro-3'-methoxy-1,1'-biphenyl-4-yl)amino]nicotinic acid, tromethamine salt.
Figure 6 illustrates the IR spectra of 2-[(3,5-difluoro-3'-methoxy-1,1'-biphenyl-4-yl)amino]nicotinic acid, tromethamine salt.
Figure 7 illustrates the DSC thermogram of 2-{[3'-ethoxy-3-(trifluoromethoxy)-1,1'-biphenyl-4-yl]aminolnicotinic acid, meglumine salt.
Figure 8 illustrates the DVS pattern of 2-{[3'-ethoxy-3-(trifluoromethoxy)-1,1'-biphenyl-4-yl]aminolnicotinic acid, meglumine salt.
Figure 9 illustrates the IR spectra of 2-{[3'-ethoxy-3-(trifluoromethoxy)-1,1'-biphenyl-4-yl]amino}nicotinic acid, meglumine salt.
Figure 10 illustrates the DSC thermogram of 2-[(3,5-difluoro-2-methyl-1,1'-biphenyl-4-yl)amino]nicotinic acid, meglumine salt monohydrate.
Figure 11 illustrates the DVS pattern of 2-[(3,5-difluoro-2-methyl-1,1'-biphenyl-4-yl)amino]nicotinic acid, meglumine salt monohydrate.
Figure 12 illustrates the IR spectra of 2-[(3,5-difluoro-2-methyl-1,1'-biphenyl-4-yl)amino]nicotinic acid, meglumine salt monohydrate.
Figure 13 illustrates the DSC thermogram of 2-[(3,5-difluoro-2-methyl-1,1'-biphenyl-4-yl)amino]nicotinic acid, tromethamine salt.
Figure 14 illustrates the DVS pattern of 2-[(3,5-difluoro-2-methyl-1,1'-biphenyl-4-yl)amino]nicotinic acid, tromethamine salt.
Figure 15 illustrates the IR spectra of 2-[(3,5-difluoro-2-methyl-1,1'-biphenyl-4-yl)amino]nicotinic acid, tromethamine salt.
Figure 16 illustrates the DSC thermogram of 2-[(3,5-difluoro-3'-methoxy-1,1'-biphenyl-4-yl)amino]nicotinic acid, L-arginine salt.
Figure 17 illustrates the DVS pattern of 2-[(3,5-difluoro-3'-methoxy-1,1'-biphenyl-4-yl)amino]nicotinic acid, L-arginine salt.
Figure 18 illustrates the IR spectra of 2-[(3,5-difluoro-3'-methoxy-1,1'-biphenyl-4-yl)amino]nicotinic acid, L-arginine salt.

### DETAILED DESCRIPTION OF THE INVENTION

When describing the salts, compositions and methods of the invention, the following terms have the following meanings, unless otherwise indicated.

The term "therapeutically effective amount" refers to an amount sufficient to effect treatment when administered to a patient in need of treatment.

The term "treatment" as used herein refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The term "solvate" refers to a complex or aggregate formed by one or more molecules of a solute, i.e. a salt of the invention or a pharmaceutically-acceptable salt thereof, and one or more molecules of a solvent. Such solvates are typically crystalline solids having a substantially fixed molar ratio of solute and solvent. Representative solvents include by way of example, water, ethanol, isopropanol and the like. When the solvent is water, the solvate formed is a hydrate.

Autoimmune diseases which may be prevented or treated include but are not limited to rheumatoid arthritis, psoriatic arthritis, systemic lupus erythematosus, multiple sclerosis, psoriasis, ankylosing spondilytis, Wegener's granulomatosis, polyarticular juvenile idiopathic arthritis, inflammatory bowel disease such as ulcerative colitis and Crohn's disease, Reiter's syndrome, fibromyalgia and type-1 diabetes.

Immune and inflammatory diseases which may be prevented or treated include but are not limited to asthma, COPD, respiratory distress syndrome, acute or chronic pancreatitis, graft versus-host disease, chronic sarcoidosis, transplant rejection, contact dermatitis, atopic dermatitis, allergic rhinitis, allergic conjunctivitis, Behcet syndrome, inflammatory eye conditions such as conjunctivitis and uveitis.

Destructive bone disorders which may be prevented or treated include but are not limited to osteoporosis, osteoarthritis and multiple myeloma-related bone disorder.

Malignant neoplastic diseases that may be prevented or treated include but are not limited to prostate, ovarian and brain cancer.

Angiogenesis-related disorders that may be prevented or treated include but are not limited to hemangiomas, ocular neovascularization, macular degeneration or diabetic retinopathy.

Viral diseases which may be prevented or treated include but are not limited to HIV infection, hepatitis and cytomegalovirus infection.

Infectious diseases which may be prevented or treated include but are not limited to sepsis, septic shock, endotoxic shock, Gram negative sepsis, toxic shock syndrome, Shigellosis and other protozoal infestations such as malaria.

Unless otherwise stated, the term alkyl embraces optionally substituted, linear or branched hydrocarbon radicals having 1 to 4 carbon atoms, preferably 1 to 2 carbon atoms. Preferred substituents on the alkyl groups are halogen atoms and hydroxy groups, more preferably halogen atoms. Alkyl groups are preferably unsubstituted, substituted with 1 or 2 hydroxy groups, or are perhaloalkyl groups. More preferably, an alkyl group is an unsubstituted alkyl group or a perhaloalkyl group. A perhaloalkyl group is an alkyl group where each hydrogen atom is replaced with a halogen atom. Preferred perhaloalkyl groups are -CF₃ and -CCl₃.

Most preferably, an alkyl group is unsubstituted.

Examples of suitable alkyl groups include methyl, ethyl, *n*-propyl, *i*-propyl, *n*-butyl, sec-butyl and *tert*-butyl radicals. Methyl is preferred. Unsubstituted methyl is most preferred.

As used herein the term alkoxy embraces optionally substituted, linear or branched oxy-containing radicals each having 1 to 4 carbon atoms, preferably 1 to 2 carbon atoms. Preferred substituents on the alkoxy groups are halogen atoms and hydroxy groups, more preferably halogen atoms. Alkoxy groups are preferably unsubstituted, substituted with 1 or 2 hydroxy groups or are perhaloalkoxy groups. More preferably, an alkoxy group is an unsubstituted alkoxy group or a perhaloalkoxy group. A perhaloalkoxy group is an alkoxy group where each hydrogen atom is replaced with a halogen atom. Preferred perhaloalkoxy groups are -OCF₃ and -OCCl₃.

Most preferably, an alkoxy group is a said perhaloalkoxy group.

Examples of suitable alkoxy groups include methoxy, ethoxy, *n*-propoxy, *i*-propoxy, n-butoxy, *sec*-butoxy and *tert*-butoxy radicals. Methoxy is preferred. Trihalomethoxy is more preferred. Trifluoromethoxy is most preferred.

As used herein, the alkyl groups or the alkoxy groups present in the general structures of the invention are "optionally substituted". This means that these alkyl or alkoxy groups can be either unsubstituted or substituted in any position by one or more, for example 1, 2, or 3 of the above substituents. When two or more substituents are present, each substituent may be the same or different.

As used herein, the term halogen atom embraces chlorine, fluorine, bromine or iodine atoms typically a fluorine, chlorine or bromine atom, most preferably bromine or fluorine. The term halo when used as a prefix has the same meaning.

In an embodiment of the present invention, in the amino nicotinic acid derivative of formula (I) R^{a} represents a group selected from halogen atoms and C₁₋₄ alkoxy groups, which may be optionally substituted by 1, 2 or 3 substituents selected from halogen atoms and hydroxy groups. Preferably, R^{a} represents a group selected from halogen atoms and C₁₋₄ alkoxy groups, which may be optionally substituted by 1, 2 or 3 halogen atoms. More preferably, R^{a} represents a group selected from halogen atoms and C₁₋₂ alkoxy groups, which are substituted by 1, 2 or 3 halogen atoms. Most preferably, R^{a} represents a group selected from halogen atoms and trihalomethoxy groups.

In another embodiment of the present invention, in the amino nicotinic acid derivative of formula (I) R^{b} represents a group selected from hydrogen atoms and halogen atoms.

In still another embodiment of the present invention, in the amino nicotinic acid derivative of formula (I) R^{c} represents a group selected from hydrogen atoms and C₁₋₄ alkyl groups, which may be optionally substituted by 1, 2 or 3 substituents selected from halogen atoms and hydroxy groups. Preferably, R^{c} represents a group selected from hydrogen atoms and C₁₋₄ alkyl groups, which may be optionally substituted by 1, 2 or 3 halogen atoms. More preferably, R^{c} represents a group selected from hydrogen atoms and C₁₋₂ alkyl groups, which may be optionally substituted by 1, 2 or 3 halogen atoms. Most preferably, R^{c} represents a group selected from hydrogen atoms and unsubstituted C₁₋₂ alkyl groups.

In yet another embodiment of the present invention, in the amino nicotinic acid derivative of formula (I) R^{d} represents a group selected from hydrogen atoms and C₁₋₄ alkoxy groups, which may be optionally substituted by 1, 2 or 3 substituents selected from halogen atoms and hydroxy groups. Preferably, R^{d} represents a group selected from hydrogen atoms and C₁₋₄ alkoxy groups, which may be optionally substituted by 1, 2 or 3 substituents selected from halogen atoms. More preferably, R^{d} represents a group selected from hydrogen atoms and C₁₋₂ alkoxy groups, which may be optionally substituted by 1, 2 or 3 halogen substituents. Most preferably, R^{d} represents a group selected from hydrogen atoms and unsubstituted C₁₋₂ alkoxy groups.

As used herein, an "amine containing one or more hydroxyl and/or carboxylic groups" is typically a straight or branched C₁-C₁₀ alkane which is substituted with one or more hydroxyl groups and/or carboxylic groups and one or more groups -NRaRb, wherein Ra and Rb are independently chosen from hydrogen atoms and straight or branched C₁-C₄ alkyl groups, which alkyl groups are unsubstituted or substituted with one or more hydroxyl groups, or Ra and Rb and the nitrogen atom to which they are bonded form a 4-to 8-membered N-containing heterocyclic ring.

Said 4- to 8-membered N-containing heterocyclic ring is typically a non aromatic saturated or unsaturated ring containing the N atom to which Ra and Rb are attached and 0, 1 or 2 further heteroatoms selected from N, O and S. Preferably, it is a 5- to 6-membered saturated ring containing 0 or 1 said further heteroatom. Typically, said further heteroatom is an O atom. Preferably, the N-containing heterocyclic ring is a morpholine of pyrrolidine ring.

Typically, the C₁-C₁₀ alkane is a C₁-C₈ alkane, preferably a C₂-C₈ alkane, more preferably a C₂-C₆ alkane, most preferably a C₄-C₆ alkane.

Typically, the C₁-C₁₀ alkane is substituted with 1, 2, 3, 4, 5, 6, 7 or 8 hydroxyl groups, preferably 1, 2, 3, 4, 5, or 6 hydroxyl groups, more preferably 1, 2, 3, 4 or 5 hydroxyl groups, most preferably 3, 4, or 5 hydroxyl groups.

Typically, the C₁-C₁₀ alkane is substituted with 1 or 2 carboxylic groups, preferably one carboxylic group.

Typically, the C₁-C₁₀ alkane is substituted with 1, 2, 3 or 4 groups -NRaRb, preferably one -NRaRb group.

Typically, the C₁-C₄ alkyl groups present as Ra or Rb are unsubstituted or substituted with 1 hydroxyl group. Preferably, the C₁-C₄ alkyl groups present as Ra or Rb are unsubstituted.

Typically, the C₁-C₄ alkyl groups present as Ra or Rb are C₁-C₂ alkyl groups, preferably methyl groups.

Preferably, Ra is a hydrogen atom and Rb is chosen from hydrogen atoms and unsubstituted methyl.

In a preferred embodiment, the "amine containing one or more hydroxyl and/or carboxylic groups" is a straight or branched C₂-C₆ alkane which is substituted with 1, 2, 3, 4, or 5 hydroxyl groups and 1 group -NRaRb, wherein Ra and Rb are independently chosen from hydrogen atoms and C₁-C₂ alkyl groups, which alkyl groups are unsubstituted or substituted with 1 hydroxyl group, or Ra and Rb and the nitrogen atom to which they are bonded form a 5 to 6 membered N-containing heterocyclic ring.

In a more preferred embodiment, the "amine containing one or more hydroxyl and/or carboxylic groups" is a straight or branched C₂-C₆ alkane which is substituted with 1, 2, 3, 4, or 5 hydroxyl groups and one group -NRaRb, wherein Ra and Rb are independently chosen from hydrogen atoms and C₁-C₂ alkyl groups, which alkyl groups are unsubstituted or substituted with 1 hydroxyl group.

In a most preferred embodiment, the "amine containing one or more hydroxyl and/or carboxylic groups" is a straight or branched C₄-C₆ alkane which is substituted with 3, 4, or 5 hydroxyl groups and one group -NRaRb, wherein Ra is hydrogen and Rb is chosen from a hydrogen atom and an unsubstituted methyl group.

In another embodiment, the "amine containing one or more hydroxyl and/or carboxylic groups" is a straight or branched C₄-C₆ alkane which is substituted with 1 or 2 carboxylic groups, and 3 or 4 groups -NRaRb, wherein Ra is hydrogen and Rb is chosen from a hydrogen atom and an unsubstituted methyl group.

In still another embodiment of the present invention, the amine containing one or more hydroxyl and/or carboxylic groups is selected from the group consisting of L-arginine, deanol (2-(dimethylamino)ethanol), diethanolamine (2,2'-iminobis(ethanol)), diethylethanolamine (2-(diethylamino)-ethanol), ethanolamine (2-aminoethanol), meglumine (N-methyl-glucamine), 2-morpholine ethanol (4-(2-hydroxyethyl)-morpholine), 1-(2-hydroxyethyl)-pyrrolidine, triethanolamine (2,2',2"-nitrilotris(ethanol)) and tromethamine (2-amino-2-(hydroxymethyl)propane-1,3-diol).

In a preferred embodiment of the present invention the amine containing one or more hydroxyl and/or carboxylic groups only contains one or more hydroxyl groups, i.e. does not contain carboxylic groups. Said amine containing one or more hydroxyl groups is preferably selected from the group consisting of deanol (2-(dimethylamino)ethanol), diethanolamine (2,2'-iminobis(ethanol)), diethylethanolamine (2-(diethylamino)-ethanol), ethanolamine (2-aminoethanol), meglumine (N-methyl-glucamine), 2-morpholine ethanol (4-(2-hydroxyethyl)-morpholine), 1-(2-hydroxyethyl)-pyrrolidine, triethanolamine (2,2',2"-nitrilotris(ethanol)) and tromethamine (2-amino-2-(hydroxymethyl)propane-1,3-diol).

In a preferred embodiment of the present invention the amine only containing one or more hydroxyl groups is selected from the group consisting of diethanolamine, meglumine, triethanolamine and tromethamine, preferably selected from the group consisting of meglumine and tromethamine.

In a preferred embodiment of the present invention the amine containing one or more hydroxyl and/or carboxylic groups only contains one or more carboxylic groups, i.e. does not contain hydroxyl groups. Preferably, said amine containing one or more carboxylic groups, preferably one carboxylic group, is L-arginine.

In a preferred embodiment of the present invention in the amino nicotinic acid derivative of formula (I) R^{a} represents a group selected from halogen atoms and C₁₋₄ alkoxy groups, preferably a group selected from halogen atoms and C₁₋₂ alkoxy groups which may be optionally substituted by 1, 2 or 3 substituents selected from halogen atoms and hydroxy groups; R^{b} represents a group selected from hydrogen atoms and halogen atoms, R^{c} represents a group selected from hydrogen atoms and C₁₋₄ alkyl groups, preferably a group selected from halogen atoms and C₁₋₂ alkyl groups, which may be optionally substituted by 1, 2 or 3 substituents selected from halogen atoms and hydroxy groups; and R^{d} represents a group selected from hydrogen atoms and C₁₋₄ alkoxy groups, preferably a group selected from halogen atoms and C₁₋₂ alkoxy groups, which may be optionally substituted by 1, 2 or 3 substituents selected from halogen atoms and hydroxy groups.

In a preferred embodiment of the present invention the amino nicotinic acid derivative of formula (I) is selected from the group consisting of 2-{[3'-ethoxy-3-(trifluoromethoxy)-1,1'-biphenyl-4-yl]amino}nicotinic acid (2-(3'-Ethoxy-3-(trifluoromethoxy)biphenyl-4-ylamino)nicotinic acid), 2-[(3,5-difluoro-2-methyl-1,1'-biphenyl-4-yl)amino]nicotinic acid (2-(3,5-difluoro-2-methylbiphenyl-4-ylamino)nicotinic acid) and 2-[(3,5-difluoro-3'-methoxy-1,1'-biphenyl-4-yl)amino]nicotinic acid (2-(3'-Ethoxy-3,5-difluorobiphenyl-4-ylamino)nicotinic acid).

In a preferred embodiment of the present invention the amine containing one or more hydroxyl and/or carboxylic groups, preferably the amine only containing one or more hydroxyl groups is selected from the group consisting of meglumine and tromethamine and in the amino nicotinic acid derivative of formula (I) R^{a} represents a group selected from halogen atoms and C₁₋₂ alkoxy groups which may be optionally substituted by 1, 2 or 3 halogen atoms; R^{b} represents a group selected from hydrogen atoms and halogen atoms, R^{c} represents a group selected from hydrogen atoms and C₁₋₂ alkyl groups which may be optionally substituted by 1, 2 or 3 substituents selected from halogen atoms and hydroxy groups; and R^{d} represents a group selected from hydrogen atoms and C₁₋₂ alkoxy groups which may be optionally substituted by 1, 2 or 3 substituents selected from halogen atoms and hydroxy groups.

In a preferred embodiment of the present invention the amine containing one or more hydroxyl and/or carboxylic groups, preferably the amine only containing one or more carboxylic groups, preferably one carboxylic group, is L-arginine and in the amino nicotinic acid derivative of formula (I) R^{a} represents a group selected from halogen atoms and C₁₋₂ alkoxy groups which may be optionally substituted by 1, 2 or 3 halogen atoms; R^{b} represents a group selected from hydrogen atoms and halogen atoms, R^{c} represents a group selected from hydrogen atoms and C₁₋₂ alkyl groups which may be optionally substituted by 1, 2 or 3 substituents selected from halogen atoms and hydroxy groups; and R^{d} represents a group selected from hydrogen atoms and C₁₋₂ alkoxy groups which may be optionally substituted by 1, 2 or 3 substituents selected from halogen atoms and hydroxy groups.

Of particular interest are the salts:
2-{[3'-ethoxy-3-(trifiuoromethoxy)-1,1'-biphenyl-4-yl]amino}nicotinic acid, meglumine salt,
2-[(3,5-difluoro-2-methyl-1,1'-biphenyl-4-yl)amino]nicotinic acid, meglumine salt,
2-[(3,5-difluoro-2-methyl-1,1'-biphenyl-4-yl)amino]nicotinic acid, tromethamine salt,
2-[(3,5-difluoro-3'-methoxy-1,1'-biphenyl-4-yl)amino]nicotinic acid, meglumine salt,
2-[(3,5-difluoro-3'-methoxy-1,1'-biphenyl-4-yl)amino]nicotinic acid, tromethamine salt,
2-[(3,5-difluoro-3'-methoxy-1,1'-biphenyl-4-yl)amino]nicotinic acid, L-arginine salt,
and pharmaceutically acceptable solvates thereof.

Typically, the crystalline salt of the invention of meglumine and 2-{[3'-ethoxy-3-(trifluoromethoxy)-1,1'-biphenyl-4-yl]amino}nicotinic acid corresponds to formula (II)

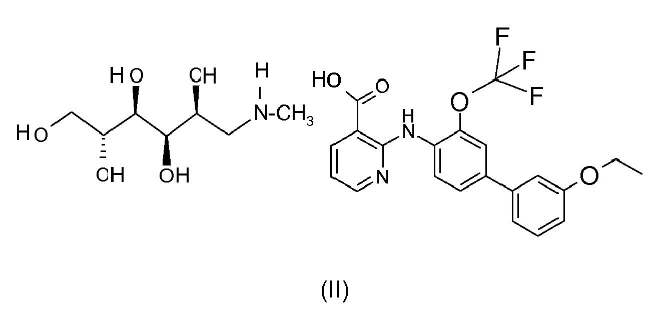

Typically, the crystalline salt of the invention of meglumine and 2-[(3,5-difluoro-2-methyl-1,1'-biphenyl-4-yl)amino]nicotinic acid of the invention corresponds to formula (III)

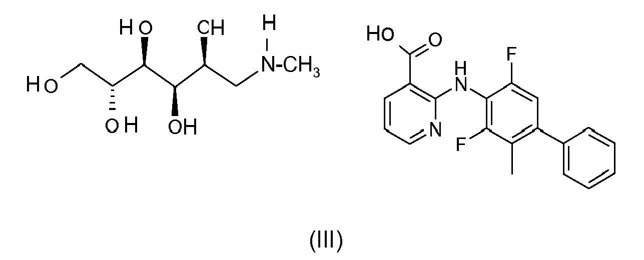

Typically, the crystalline salt of the invention of tromethamine and 2-[(3,5-difluoro-2-methyl-1,1'-biphenyl-4-yl)amino]nicotinic acid of the invention corresponds to formula (IV)

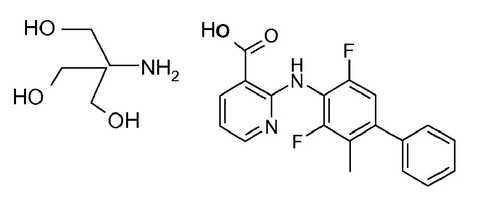

Typically, the crystalline salt of the invention of meglumine and 2-[(3,5-difluoro-3'-methoxy-1,1'-biphenyl-4-yl)amino]nicotinic acid of the invention corresponds to formula (V)

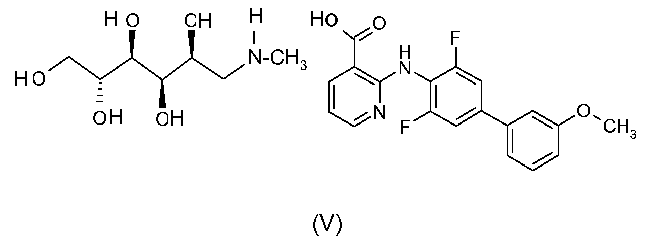

Typically, the crystalline salt of the invention of tromethamine and 2-[(3,5-difluoro-3'-methoxy-1,1'-biphenyl-4-yl)amino]nicotinic acid of the invention corresponds to formula (VI)

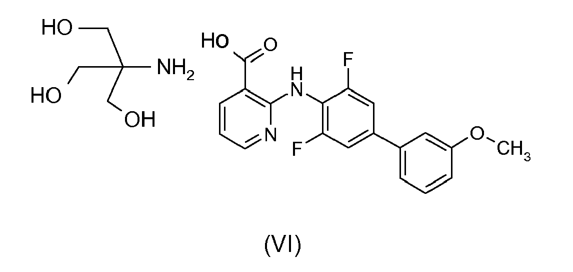

Typically, the crystalline salt of the invention of L-arginine and 2-[(3,5-difluoro-3'-methoxy-1,1'-biphenyl-4-yl)amino]nicotinic acid of the invention corresponds to formula (VII)

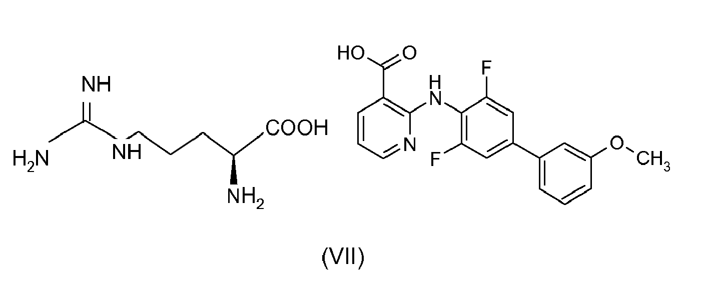

The invention also encompasses pharmaceutical compositions comprising a therapeutically effective amount of a salt as hereinabove defined and a pharmaceutically acceptable carrier.

In an embodiment of the present invention the pharmaceutical composition further comprises a therapeutically effective amount of one or more other therapeutic agents.

The invention is also directed to combinations comprising a salt of the invention and one or more other therapeutic agents and pharmaceutical compositions comprising such combinations.

The invention is also directed to a salt of the invention as described herein, a combination of a salt of the invention together with one or more other therapeutic agents or a pharmaceutical composition comprising such combination for use in the treatment of a pathological condition or disease susceptible to amelioration by inhibition of dihydroorotate dehydrogenase, in particular wherein the pathological condition or disease is selected from rheumatoid arthritis, psoriatic arthritis, ankylosing spondilytis, multiple sclerosis, Wegener's granulomatosis, systemic lupus erythematosus, psoriasis and sarcoidosis. The invention also encompasses the use of the salt of the invention, a combination of a salt of the invention together with one or more other therapeutic agents or a pharmaceutical composition comprising such combination for the manufacture of a formulation or medicament for treating these diseases.

The invention also encompasses a method of treatment of a pathological condition or disease susceptible to amelioration by inhibition of dihydroorotate dehydrogenase, in particular wherein the pathological condition or disease is selected from rheumatoid arthritis, psoriatic arthritis, ankylosing spondilytis, multiple sclerosis, Wegener's granulomatosis, systemic lupus erythematosus, psoriasis and sarcoidosis, comprising administering a therapeutically effective amount of a salt of the invention. The invention also encompasses a method of treatment comprising administering a therapeutically effective amount of a combination of a salt of the invention together with one or more other therapeutic agents or administering a therapeutically effective amount of a pharmaceutical composition comprising such combination.

### General Synthetic Procedures

The salts of the invention can be prepared using the methods and procedures described herein, or using similar methods and procedures. It will be appreciated that where typical or preferred process conditions (i.e., reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Processes for preparing salts of the invention are provided as further embodiments of the invention and are illustrated by the procedures below.

The salts of the invention can be synthesized from the corresponding amino nicotinic acid derivative of formula (I) and from the amine containing one or more hydroxyl groups, which are commercially available from, for example, Aldrich.

Suitable inert diluents for this reaction include, but are not limited to, acetone, ethyl acetate, N,N-dimethylformamide, chloroform, methanol, ethanol, isopropanol, 2-butanol, acetonitrile, dimethyl carbonate, nitromethane and the like, and mixtures thereof, optionally containing water.

Upon completion of any of the foregoing reactions, the salt can be isolated from the reaction mixture by any conventional means such as precipitation, concentration, centrifugation and the like.

It will be appreciated that while specific process conditions (i.e. reaction temperatures, times, mole ratios of reactants, solvents, pressures, etc.) are given, other process conditions can also be used unless otherwise stated.

A water-soluble salt of the invention typically contains between about 0.60 and 1.20 molar equivalents of amino nicotinic acid derivative of formula (I) per molar equivalent of the free base, more typically 0.85 and 1.15 molar equivalents of amino nicotinic acid derivative of formula (I) per molar equivalent of the free base, even more typically about 1 molar equivalent of amino nicotinic acid derivative of formula (I) per molar equivalent of the free base.

The molar ratios described in the methods of the invention can be readily determined by various methods available to those skilled in the art. For example, such molar ratios can be readily determined by ¹H NMR. Alternatively, elemental analysis and HPLC methods can be used to determine the molar ratio.

### EXAMPLES

General. Reagents, starting materials, and solvents were purchased from commercial suppliers and used as received.

Crystallization tests of salts of some amino nicotinic acid derivatives of formula (I) with a broad range of pharmaceutically acceptable acids (comprising among others hydrochloric, methanesulfonic and sulphuric acids) and bases (comprising among others ammonia, L-arginine, choline, magnesium hydroxide, meglumine, potassium hydroxide, sodium hydroxide and tromethamine) in a range of different pharmaceutically acceptable solvents (including among others acetone, acetonitrile, ethyl acetate, isobutyl acetate, chloroform, nitromethane, dimethyl carbonate, N,N-dimethylformamide, ethanol, isopropanol and methanol) have been undertaken.

The salts from ammonia, choline, and magnesium hydroxide rendered either oils or amorphous solids. The salts from potassium hydroxide and sodium hydroxide were hygroscopic. On the other hand, the salts from hydrochloric acid, methanesulfonic acid and sulfuric acid had similar or even lower solubility than the free acid form. In addition to this low solubility, the salts of these acids manifested a very bad humectability in water.

Only the salts of the invention were neither hygroscopic nor deliquescent and had a relatively high melting point allowing them to be micronized and to have long term stability.

Particularly good methods to prepare the addition salts of the invention are illustrated in the following examples.

The differential scanning calorimetry (DSC) thermograms analyses were obtained using a DSC-821 Mettler-Toledo instrument, serial number 5117423874. Samples were weighed into an aluminium pan, an aluminium lid placed on top of the sample and compressed with a brass rod. Samples were equilibrated at 30°C and heated at 10°C / min to 350°C. The instrument was calibrated using indium and zinc standards.

Infrared spectroscopy (IR) spectra were obtained using a Perkin Elmer Spectrum One FT-IR instrument, serial number 70749, equipped with a universal ATR accessory. Solid samples were introduced directly into the ATR. The acquisition range was 650 to 4000 cm⁻¹.

Dynamic Vapour Sorption (DVS) profiles were obtained using an Igasorp Hiden Isochema instrument (serial number IGA-SA-066). After an initial stabilization period, at least two isotherms (at 25°C) were obtained for each sample: a moisture sorption from 0 to 95% relative humidity and moisture desorption from 95% relative humidity to dryness. Both isotherms were performed in 10% humidity steps, with a minimum time of 10 minutes and a maximum time of 30 minutes for each step.

### Example 1: Preparation of 2-[(3,5-difluoro-3'-methoxy-1,1'-biphenyl-4-yl)amino]nicotinic acid, meglumine salt

275 mg (1.4 mmol) of meglumine were dissolved in 30 mL of methanol and said solution was added to a mixture of 500 mg (1.4 mmol) of 2-[(3,5-difluoro-3'-methoxy-1,1'-biphenyl-4-yl)amino]nicotinic acid dissolved in the minimum amount of tetrahydrofurane (7 mL). After 30 min of stirring at room temperature, the solution was evaporated under vacuum. The precipitate formed was suspended in 20 mL of ethyl acetate at 77°C during 30 min, the resulting suspension was then filtered and the obtained precipitate was dried under vacuum for 4 hours at 40°C. 675 mg (yield: 87%) of a white solid was then obtained with a purity of 99.9% by HPLC.

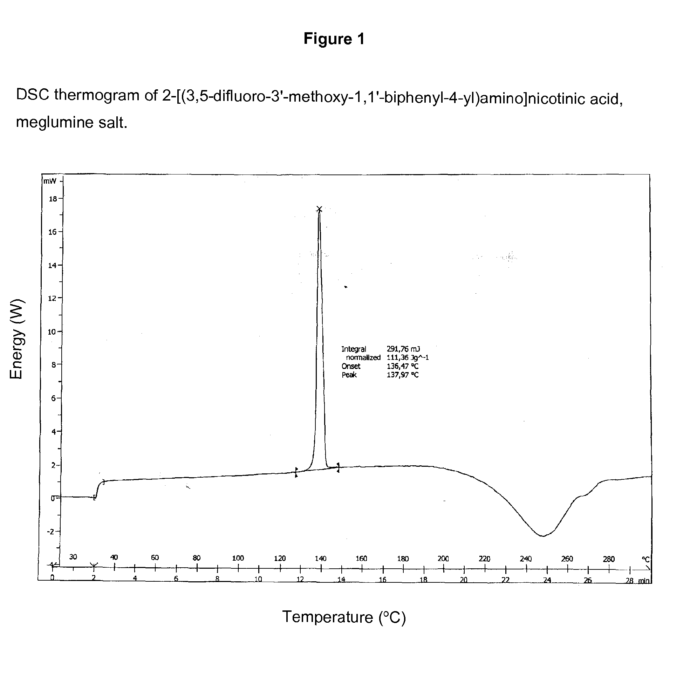

Figure 1 illustrates the DSC thermogram of the meglumine salt of 2-[(3,5-difluoro-3'-methoxy-1,1'-biphenyl-4-yl)amino]nicotinic acid. The sample exhibits a characteristic high endotherm at onset 136°C that corresponds to a melting or decomposition of the salt. This indicates that the sample does not convert into any other polymorphs and does not suffer any decomposition, confirming thus its high stability.

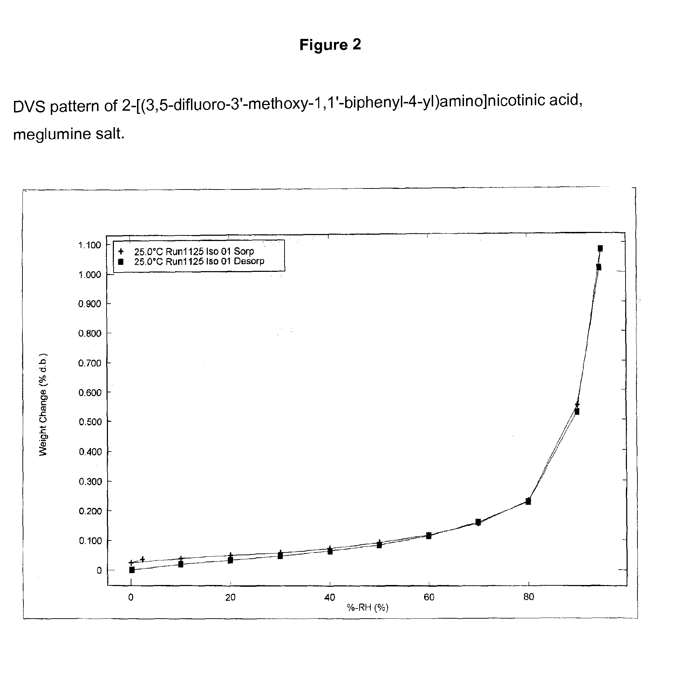

Figure 2 illustrates the DVS pattern of the meglumine salt of 2-[(3,5-difluoro-3'-methoxy-1,1'-biphenyl-4-yl)amino]nicotinic acid. Mass increase was measured at 80% (0.15% increase) and 90% (0.4% increase) relative humidity (RH). According to the results, said salt is not hygroscopic and exhibited no hysteresis.

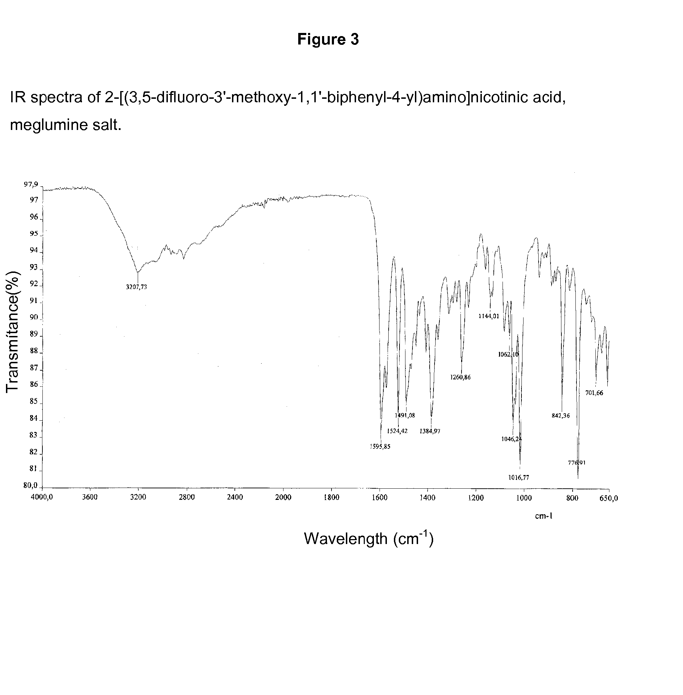

Figure 3 illustrates the IR spectra of the meglumine salt of 2-[(3,5-difluoro-3'-methoxy-1,1'-biphenyl-4-yl)amino]nicotinic acid. Characteristic signals appear at 3207, 1595, 1524, 1491, 1384, 1260, 1144, 1062, 1046, 1016, 842, 776 and 701 cm-1.

### Example 2: Preparation of 2-[(3,5-difluoro-3'-methoxy-1,1'-biphenyl-4-yl)amino]nicotinic acid, tromethamine salt

170 mg (1.4 mmol) of tromethamine were dissolved in 20 mL of methanol and said solution was added to a mixture of 500 mg (1.4 mmol) of 2-[(3,5-difluoro-3'-methoxy-1,1'-biphenyl-4-yl)amino]nicotinic acid dissolved in the minimum amount of tetrahydrofurane (6 mL). After 30 min of stirring at room temperature, the solution was evaporated under vacuum. The oil obtained was dissolved in 0.9 mL of chloroform at 61°C and it was cooled slowly to room temperature. After 3 days the suspension was decanted and the obtained precipitate was dried under vacuum for 4 hours at 40°C. 665 mg (yield: 99%) of a white solid was then obtained with a purity of 99.9% by HPLC.

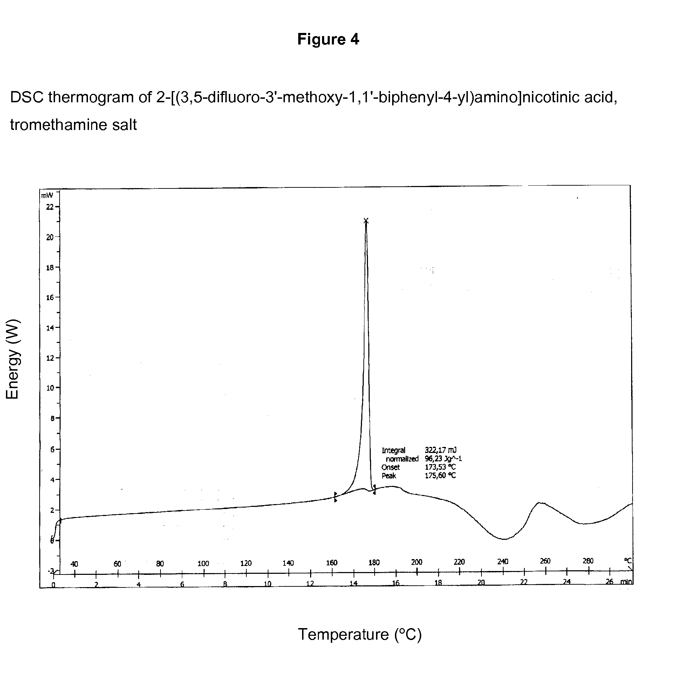

Figure 4 illustrates the DSC thermogram of the tromethamine salt of 2-[(3,5-difluoro-3'-methoxy-1,1'-biphenyl-4-yl)amino]nicotinic acid. The sample exhibits a characteristic high endotherm at onset 173°C that corresponds to a melting or decomposition of the salt. This indicates that the sample does not convert into any other polymorphs and does not suffer any decomposition, confirming thus its high stability.

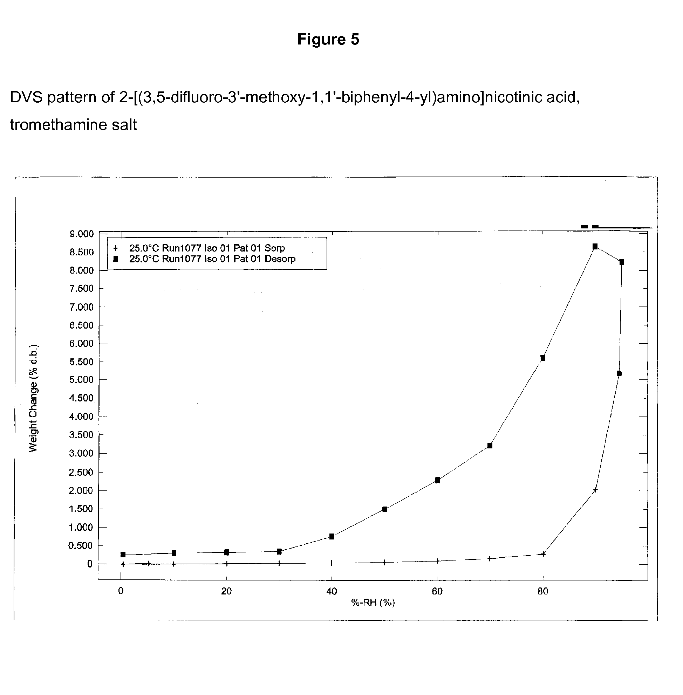

Figure 5 illustrates the DVS pattern of the tromethamine salt of 2-[(3,5-difluoro-3'-methoxy-1,1'-biphenyl-4-yl)amino]nicotinic acid. Mass increase was measured at 80% (0.1% increase) and 95% (2.0% increase) relative humidity (RH). According to the result, said salt is not hygroscopic and exhibited no hysteresis.

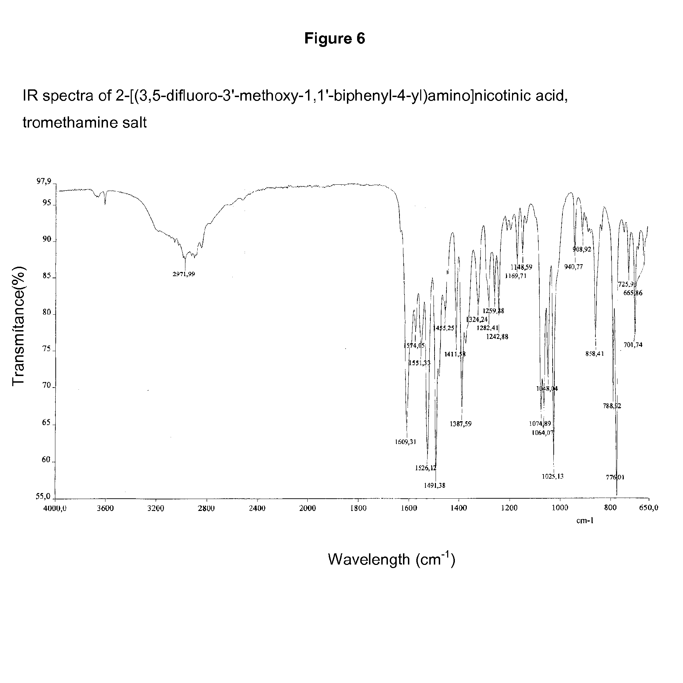

Figure 6 illustrates the IR spectra of the tromethamine salt of 2-[(3,5-difluoro-3'-methoxy-1,1'-biphenyl-4-yl)amino]nicotinic acid. Characteristic signals appear at 2971, 1609, 1574, 1551, 1526, 1491, 1455, 1411, 1387, 1324, 1282, 1259, 1242, 1169, 1148, 1064, 1025, 858 and 776 cm-1.

### Example 3: Preparation of 2-(3'-ethoxy-3-(trifluoromethoxy)biphenyl-4-ylamino) nicotinic acid, meglumine salt

26 mg (0.133 mmol) of meglumine were added to a mixture of 90 mg (0.215 mmol) of 2-(3'-ethoxy-3-(trifluoromethoxy)biphenyl-4-ylamino) nicotinic acid dissolved in the minimum amount of tetrahydrofurane (9 mL). After 30 min of stirring at room temperature, the solution was evaporated under vacuum. The precipitate formed was suspended in 18 mL of ethyl acetate at 77°C during 30 min. The resulting suspension was then cooled to 20 °C for a minimum of 1 hour, cooled to 0-5 °C for 1 hour, filtered and the obtained precipitate was dried under vacuum for 4 hours at 50°C. 70 mg (yield: 53%) of a white solid was then obtained with a purity of 100% by HPLC.

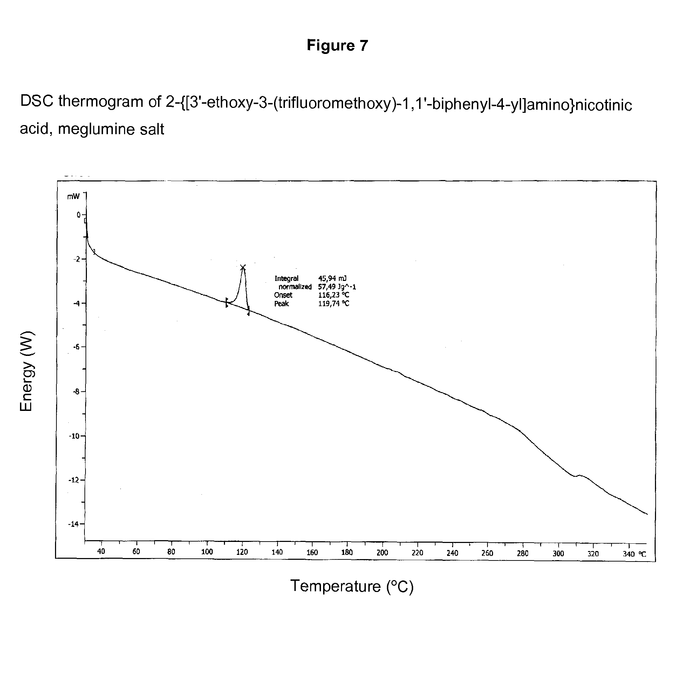

Figure 7 illustrates the DSC thermogram of the meglumine salt of 2-(3'-ethoxy-3-(trifluoromethoxy)biphenyl-4-ylamino) nicotinic acid. The sample exhibits a characteristic endotherm at onset 116°C that corresponds to a melting or decomposition of the salt. This indicates that the sample does not convert into any other polymorphs and does not suffer any decomposition, confirming thus its high stability.

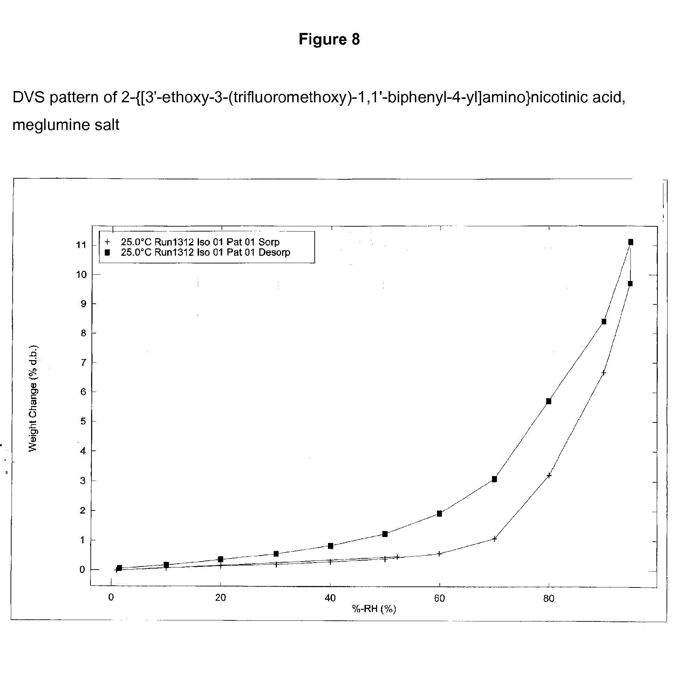

Figure 8 illustrates the DVS pattern of the meglumine salt of 2-(3'-ethoxy-3-(trifluoromethoxy)biphenyl-4-ylamino) nicotinic acid. Mass increase was measured at 80% (2.7% increase) and 90% (6.2% increase) relative humidity (RH). According to the results, said salt is slightly hygroscopic and exhibited no hysteresis.

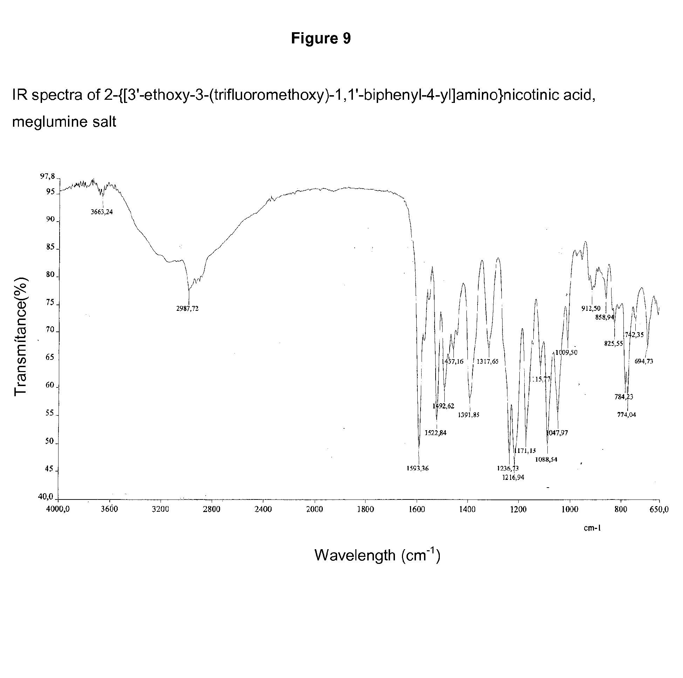

Figure 9 illustrates the IR spectra of the meglumine salt of 2-(3'-ethoxy-3-(trifluoromethoxy)biphenyl-4-ylamino) nicotinic acid. Characteristic signals appear at 3663, 2987,1593,1522,1492,1457,1391,1317,1236,1216,1171,1088,1062,1047,1009, 858, 777 and 694 cm-1.

### Example 4: Preparation of 2-(3,5-difluoro-2-methylbiphenyl-4-ylamino)nicotinic acid, meglumine salt

32 mg (0.164 mmol) of meglumine were added to a mixture of 90 mg (0.265 mmol) of 2-(3,5-difluoro-2-methylbiphenyl-4-ylamino)nicotinic acid dissolved in the minimum amount of tetrahydrofurane (9 mL). After 30 min of stirring at room temperature, the solution was evaporated under vacuum. The precipitate formed was suspended in 4 mL of *t-*butylmethyl ether and 2 mL of methylene chloride and stirred at 20°C until a complete dissolution was observed. The solvent was partially removed at 20°C under vacuum until precipitation of a solid was observed. The resulting suspension was then cooled to 0-5°C for a minimum of 1 hour, filtered and the obtained precipitate was dried under vacuum for 4 hours at 50°C. 58 mg (yield: 41%) of an off-white solid was then obtained with a purity of 100% by HPLC.

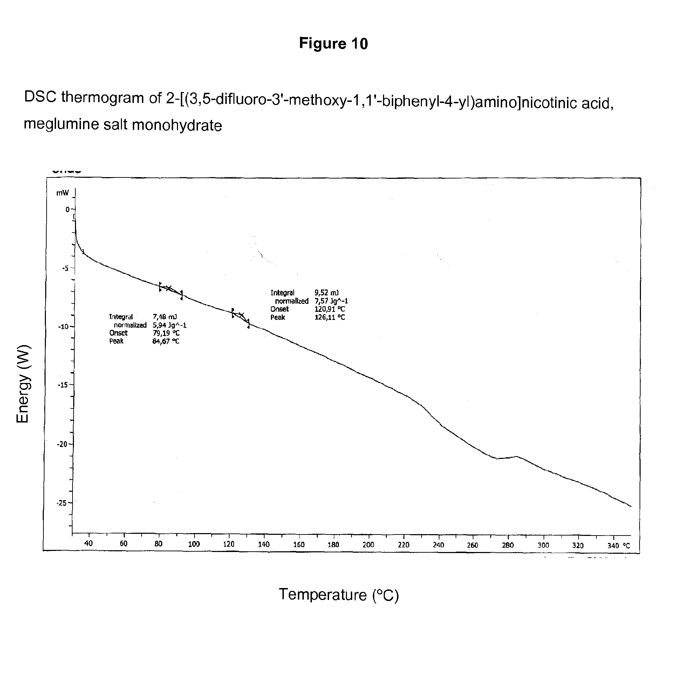

Figure 10 illustrates the DSC thermogram of the meglumine salt of 2-(3,5-difluoro-2-methylbiphenyl-4-ylamino)nicotinic acid monohydrate. The sample exhibits two small endotherms at onset 79°C and 121°C. The first one probably corresponds to the loss of water, and the second one corresponds to melting or decomposition of the anhydrous form. No other transitions are detected.

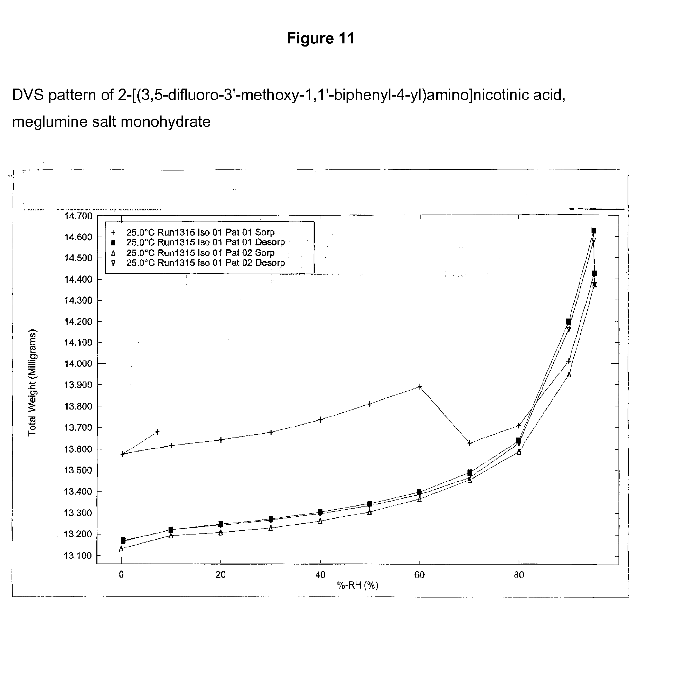

Figure 11 illustrates the DVS pattern of the meglumine salt of 2-(3,5-difluoro-2-methylbiphenyl-4-ylamino)nicotinic acid monohydrate. This DVS profile shows four isotherms (instead of the usual two), corresponding to the absorption-desorption profiles of the monohydrate and the anhydrous form. According to the results, both hydrate and anhydrous form are not hygroscopic. The anhydrous form could be obtained by drying the monohydrate.

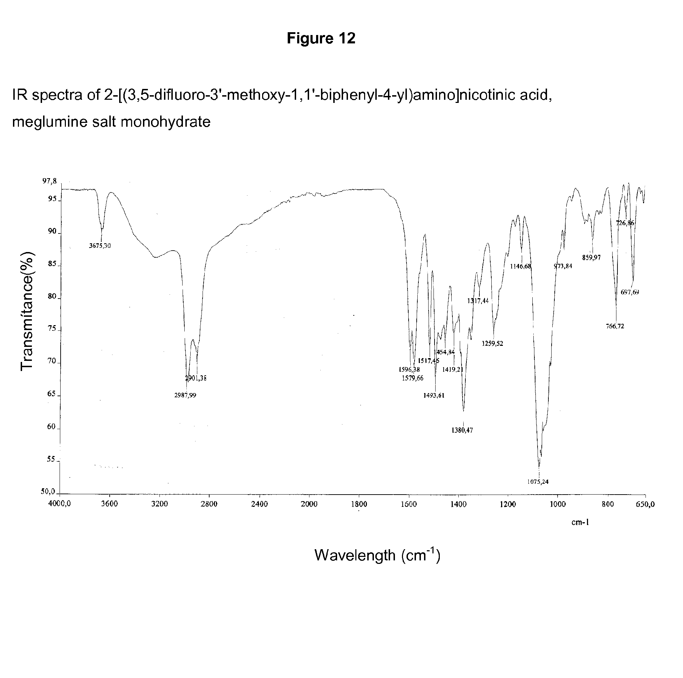

Figure 12 illustrates the IR spectra of the meglumine salt of 2-(3,5-difluoro-2-methylbiphenyl-4-ylamino)nicotinic acid monohydrate. Characteristic signals appear at 3675,2987,2901,1596,1579,1517,1493,1454,1419,1380,1317,1259,1146,1075, 973, 859, 766 and 697 cm-1.

### Example 5: Preparation of 2-(3,5-difluoro-2-methylbiphenyl-4-ylamino) nicotinic acid, tromethamine salt

31.5 mg (0.260 mmol) of tromethamine were dissolved in 25 mL of methanol and said solution was added to a mixture of 55 mg (0.161 mmol) of 2-(3,5-difluoro-2-methylbiphenyl-4-ylamino) nicotinic acid dissolved in the minimum amount of tetrahydrofurane (6.6 mL). After 30 min of stirring at room temperature, the solution was evaporated under vacuum. The oil obtained was dissolved in 4.4 mL of chloroform at 61°C and it was cooled slowly to room temperature overnight. The suspension was cooled to 0-5°C for 1 hour, filtered and the obtained precipitate was dried under vacuum for 4 hours at 50°C. 77 mg (yield: 99%) of an off-white solid was then obtained with a purity of 100% by HPLC.

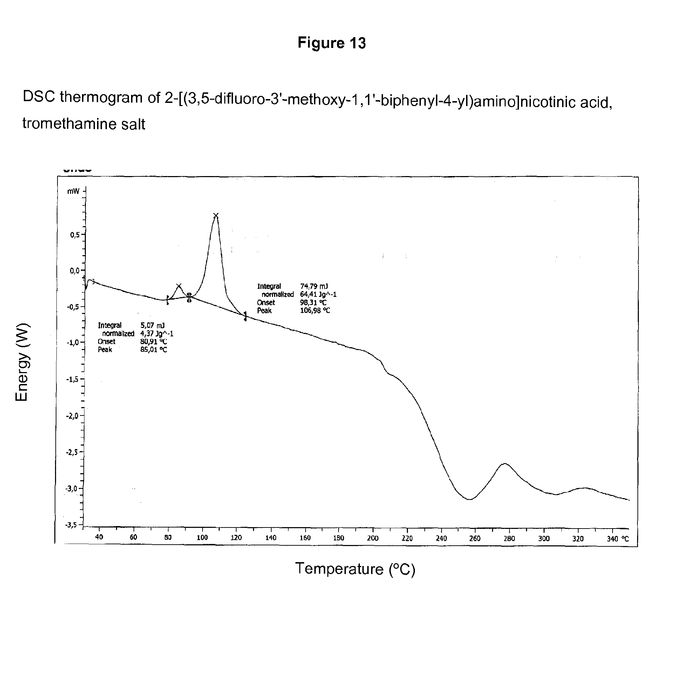

Figure 13 illustrates the DSC thermogram of the tromethamine salt of 2-(3,5-difluoro-2-methylbiphenyl-4-ylamino) nicotinic acid. The sample exhibits two endotherms at onset 81 and 98°C. The last one corresponds to a melting or decomposition of the salt.

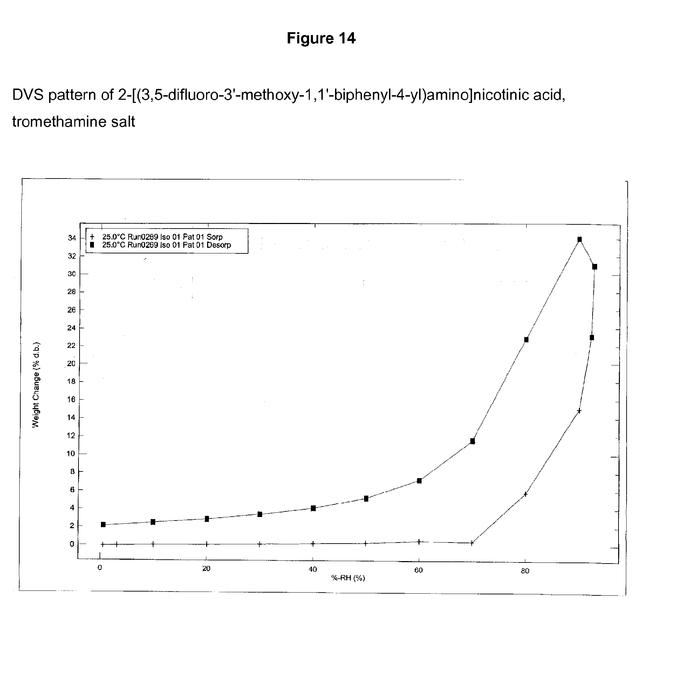

Figure 14 illustrates the DVS pattern of the tromethamine salt of 2-(3,5-difluoro-2-methylbiphenyl-4-ylamino) nicotinic acid. Mass increase was measured at 80% (5.7% increase) and 90% (14.9% increase) relative humidity (RH). According to the result, said salt is a bit hygroscopic but without hysteresis: no stable hydration is detected.

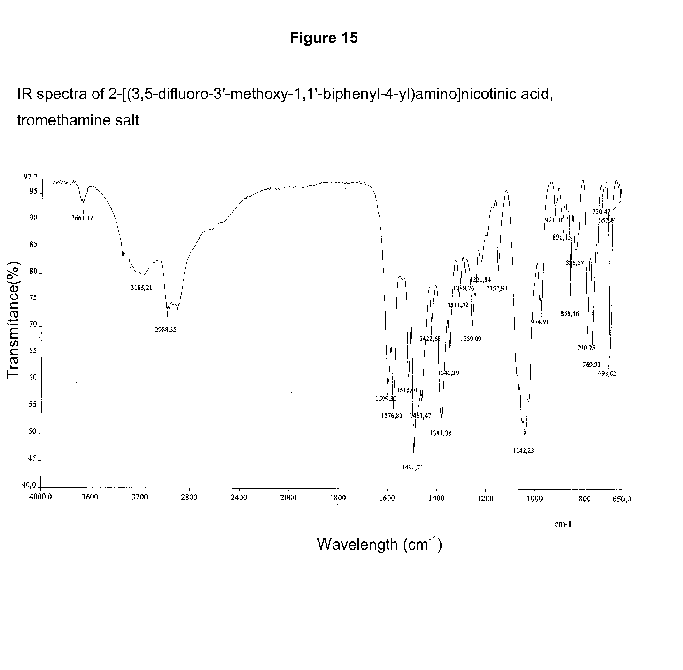

Figure 15 illustrates the IR spectra of the tromethamine salt of 2-(3,5-difluoro-2-methylbiphenyl-4-ylamino) nicotinic acid. Characteristic signals appear at 3663, 3185, 2988,1599,1576,1515,1492,1461,1422,1381,1349,1311,1288,1259,1221,1152, 1042, 974, 858 and 769 cm-1.

### Example 6: Preparation of 2-[(3,5-difluoro-3'-methoxy-1,1'-biphenyl-4-yl)amino]nicotinic acid, L-arginine salt

245 mg (1.4 mmol) of L-arginine were dissolved in 6 mL of water and said solution was added to a mixture of 500 mg (1.4 mmol) of 2-[(3,5-difluoro-3'-methoxy-1,1'-biphenyl-4-yl)amino]nicotinic acid dissolved in the minimum amount of tetrahydrofurane (7 mL). After 30 min of stirring at room temperature, the solution was evaporated under vacuum. The precipitate formed was suspended in 20 mL of methanol at 65 °C during 30 min, the resulting suspension was filtered and the obtained solid was dried under vacuum for 4 hours at 40 °C. 680 mg (yield 91 %) of a white solid was then obtained with a purity of 99.9% by HPLC.

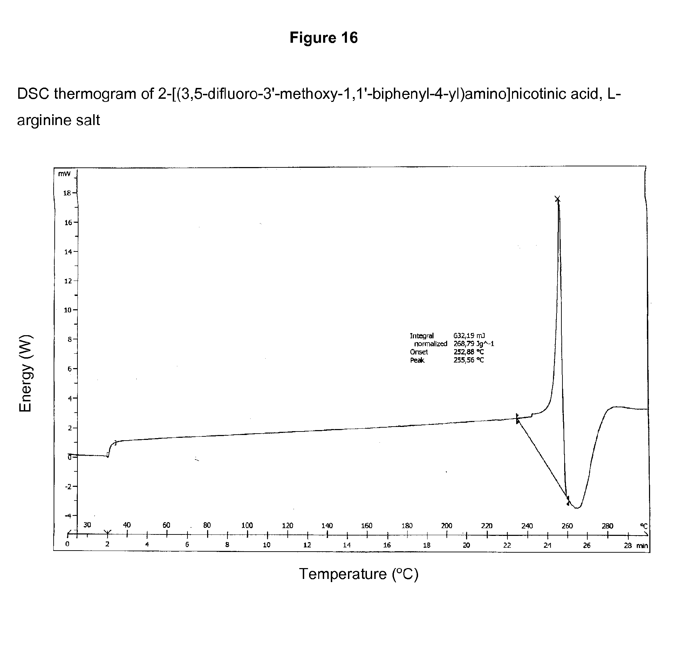

Figure 16 illustrates the DSC thermogram of the L-arginine salt of 2-[(3,5-difluoro-3'-methoxy-1,1'-biphenyl-4-yl)amino]nicotinic acid. The sample exhibits a characteristic high endotherm at onset 253°C that corresponds to a melting or decomposition of the salt. This indicates that the sample does not convert into any other polymorphs and does not suffer any decomposition, confirming thus its high stability.

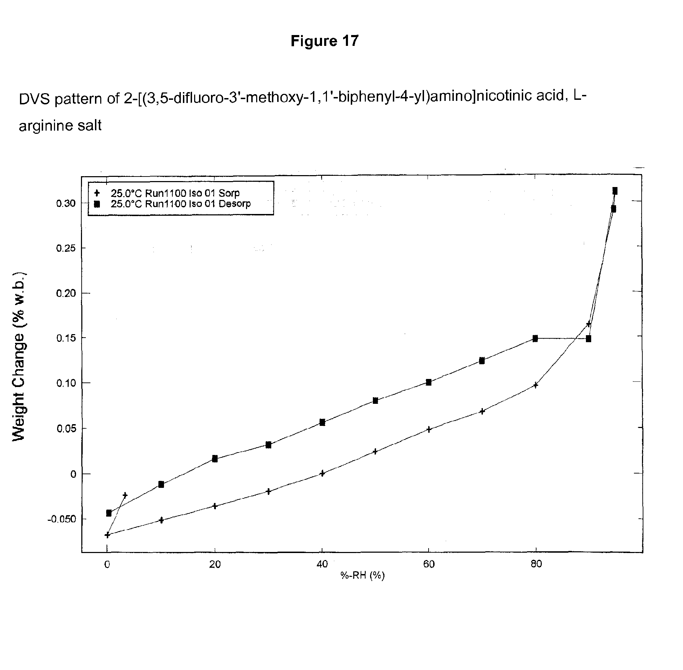

Figure 17 illustrates the DVS pattern of the L-arginine salt of 2-[(3,5-difluoro-3'-methoxy-1,1'-biphenyl-4-yl)amino]nicotinic acid. Mass increase was measured at 80% (0.1% increase) and 90% (0.30% increase) relative humidity (RH). According to the results, said salt is not hygroscopic and exhibited no hysteresis.

Figure 18 illustrates the IR spectra of the L-arginine salt of 2-[(3,5-difluoro-3'-methoxy-1,1'-biphenyl-4-yl)amino]nicotinic acid. Characteristic signals appear at 3439, 3315, 2988, 1684,1601,1575,1528,1489,1472,1453,1405,1390,1348,1318,1263,1236,1162, 1143,1044,1022,936,899,870,852,829,804,772,727, 691, and 658 cm-1.

### Water- Solubility test:

The solubility of the of Examples 1-5 in water at room temperature was determined together with the solubility of the corresponding free acids. The results are shown in Table 1 below.

| **Ex.** | **Product** | **Water Solubility** **@ 25°C. (mg/mL** **as acid)** |
|---|---|---|
| C1 | 2-[(3,5-difluoro-3'-methoxy-1,1'-biphenyl-4-yl)amino]nicotinic acid | 0.004 |
| Ex. 1 | 2-[(3,5-difluoro-3'-methoxy-1,1'-biphenyl-4-yl)amino]nicotinic acid, meglumine salt | 2.810 |
| Ex. 2 | 2-[(3,5-difluoro-3'-methoxy-1,1'-biphenyl-4-yl)amino]nicotinic acid, tromethamine salt | 0.870 |
| C2 | 2-{[3'-ethoxy-3-(trifluoromethoxy)-1,1'-biphenyl-4-yl]amino}nicotinic acid | 0.008 |
| Ex. 3 | 2-{[3'-ethoxy-3-(trifluoromethoxy)-1,1'-biphenyl-4-yl]amino}nicotinic acid, meglumine salt | 0.478 |
| C3 | 2-[(3,5-difluoro-2-methyl-1,1'-biphenyl-4-yl)amino]nicotinic acid | 0.002 |
| Ex. 4 | 2-[(3,5-difluoro-2-methyl-1,1'-biphenyl-4-yl)amino]nicotinic acid, meglumine salt | 0.210 |
| Ex. 5 | 2-[(3,5-difluoro-2-methyl-1,1'-biphenyl-4-yl)amino]nicotinic acid, tromethamine salt | 0.490 |
| Ex. 6 | 2-[(3,5-difluoro-3'-methoxy-1,1'-biphenyl-4-yl)amino]nicotinic acid, L-arginine salt | 0.690 |

As it can be seen for the table, the salts of the present invention present a higher solubility over the corresponding free acids. Particularly good results are obtained with the meglumine salt, the tromethamine salt and the L-arginine salt of 2-[(3,5-difluoro-3'-methoxy-1,1'-biphenyl-4-yl)amino]nicotinic acid (Ex. 1, Ex. 2 and Ex. 6), especially with the meglumine salt of 2-[(3,5-difluoro-3'-methoxy-1,1'-biphenyl-4-yl)amino]nicotinic acid (Ex. 1)

### Pharmaceutical Compositions

Pharmaceutical compositions according to the present invention comprise a salt of the invention or pharmaceutically acceptable solvate thereof and a pharmaceutically acceptable carrier.

The salts of the invention are useful in the treatment or prevention of diseases known to be susceptible to improvement by treatment with inhibitor of the dihydroorotate dehydrogenase. Such diseases include but are not limited to rheumatoid arthritis, psoriatic arthritis, ankylosing spondilytis, multiple sclerosis, Wegener's granulomatosis, systemic lupus erythematosus, psoriasis and sarcoidosis.

The salts of the invention may also be combined with other active compounds in the treatment of diseases known to be susceptible to improvement by treatment with an inhibitor of the dihydroorotate dehydrogenase.

The combinations of the invention can optionally comprise one or more additional active substances which are known to be useful in the treatment of autoimmune diseases, immune and inflammatory diseases, destructive bone disorders, malignant neoplastic diseases, angiogenic-related disorders, viral diseases, and infectious diseases such as (a) Anti-TNF-alpha monoclonal antibodies such as Infliximab, Certolizumab pegol, Golimumab, Adalimumab and AME-527 from Applied Molecular Evolution, (b) Antimetabolite compounds such as Mizoribine, Cyclophosphamide and Azathiopirine, (c) Calcineurin (PP-2B) Inhibitors / INS Expression Inhibitors such as cyclosporine A, Tacrolimus and ISA-247 from Isotechnika, (d) Cyclooxygenase Inhibitors such as Aceclofenac, Diclofenac, Celecoxib, Rofecoxib, Etoricoxib, Valdecoxib, Lumiracoxib, Cimicoxib and LAS-34475 from Laboratorios Almirall, S.A., (e) TNF-alpha Antagonists such as Etanercept, Lenercept, Onercept and Pegsunercept, (f) NF-kappaB (NFKB) Activation Inhibitors such as Sulfasalazine and Iguratimod, (g) IL-1 Receptor Antagonists such as Anakinra and AMG-719 from Amgen, (h) Dihydrofolate Reductase (DHFR) Inhibitors such as Methrotexate, Aminopterin and CH-1504 from Chelsea, (i) Inhibitors of Inosine 5'-Monophosphate Dehydrogenase (IMPDH) such as Mizoribine, Ribavirin, Tiazofurin, Amitivir, Mycophenolate mofetil, Ribamidine and Merimepodib, (j) Glucocorticoids such as Prednisolone, Methylprednisolone, Dexamethasone, Cortisol, Hydrocortisone, Triamcinolone acetonide, Fluocinolone acetonide, Fluocinonide, Clocortolone pivalate, Hydrocortisone aceponate, Methylprednisolone suleptanate, Betamethasone butyrate propionate, Deltacortisone, Deltadehydrocortisone, Prednisone, Dexamethasone sodium phosphate, Triamcinolone, Betamethasone valerate, Betamethasone, Hydrocortisone sodium succinate, Prednisolone sodium phosphate, Hydrocortisone probutate and Difluprednate, (k) Anti-CD20 monoclonal antibodies such as Rituximab, Ofatumumab, Ocrelizumab and TRU-015 from Trubion Pharmaceuticals, (I) B-targeted cell therapies such as BLYSS, BAFF, TACI-Ig and APRIL, (m) p38 Inhibitors such as AMG-548 (from Amgen), ARRY-797 (from Array Biopharma), Chlormethiazole edisylate, Doramapimod, PS-540446 (from BMS), SB-203580, SB-242235, SB-235699, SB-281832, SB-681323, SB-856553 (all from GlaxoSmithKline), KC-706 (from Kemia), LEO-1606, LEO-15520 (all from Leo), SC-80036, SD-06 (all from Pfizer), RWJ-67657 (from R.W. Johnson), RO-3201195, RO-4402257 (all from Roche), AVE-9940 (from Aventis), SCIO-323, SCIO-469 (all from Scios), TA-5493 (from Tanabe Seiyaku), and VX-745, VX-702 (all from Vertex) and the compounds claimed or described in Spanish patent applications numbers ES2303758 and ES2301380, (n) Jak3 Inhibitors such as CP690550 from Pfizer, (o) Syk inhibitors such as R-112, R-406 and R-788 all from Rigel, (p) MEK inhibitors such as ARRY-142886, ARRY-438162 (all from Array Biopharma), AZD-6244 (from AstraZeneca), PD-098059, PD-0325901 (all from Pfizer), (q) P2X7 receptor antagonist such as AZD-9056 from AstraZeneca, (r) S1P1 agonists such as Fingolimod, CS-0777 from Sankyo and R-3477 from Actelion, (s) Anti-CD49 monoclonal antibodies such as Natalizumab, (t) Integrin Inhibitors such as Cilengitide, Firategrast, Valategrast hydrochloride, SB-273005, SB-683698 (all from Glaxo), HMR-1031 from Sanofi-Aventis, R-1295 from Roche, BMS-587101 from BMS and CDP-323 from UCB Celltech, (u) Anti-CD88 monoclonal antibodies such as Eculizumab and Pexelizumab, (v) IL-6 receptor antagonist such as CBP-1011 from InKine and C-326 from Amgen, (w) Anti IL-6 monoclonal antibodies such as Elsilimomab, CNTO-328 from Centocor and VX-30 from Vaccinex, (x) Anti-CD152 monoclonal antibodies such as Ipilimumab and Ticilimumab, (y) Fusion proteins comprising the extracellular domain of human cytotoxic T-lymphocyte-associated antigen 4 (CTLA-4) linked to portions of human immunoglobulin G1 such as Abatacept, (z) Agents useful in the treatment of bone disorders such as Bisphophonates such as Tiludronate disodium, Clodronate disodium, Disodium pamidronate, Etidronate disodium, Xydiphone (K, Na salt), Alendronate sodium, Neridronate, Dimethyl-APD, Olpadronic acid sodium salt, Minodronic acid, Apomine, Ibandronate sodium hydrate and Risedronate sodium, (aa) VEGF Try kinase inhibitors such as Pegaptanib octasodium, Vatalanib succinate, Sorafenib, Vandetanib, Sunitinib malate, Cediranib, Pazopanib hydrochloride and AE-941 from AEterna Zentaris, (bb) Other compounds efficacious in autoimmune diseases such as Gold salts, hydroxycloroquinine, Penicilamine, K-832, SMP114 and AD452, (cc) Purine-Nucleoside phosphorylase inhibitors such as Forodesine hydrochloride, R-3421 from Albert Einstein College of Medicine, Cl-972 and Cl-1000 both from Pfizer, (dd) Anti-RANKL monoclonal antibodies such as Denosumab, (ee) Anti-CD25 monoclonal antibodies such as Inolimomab, Dacliximab, Basiliximab and LMB-2 from the US National Cancer Institute, (ff) Histone Deacetylase (HDAC) Inhibitors such as Divalproex sodium, Acetyldinaline, Depsipeptide, Sodium butyrate, Sodium phenylbutyrate, Vorinostat, MS-27-275 from Mitsui, Valproic acid, Pyroxamide, Tributyrin, PX-105684 from TopoTarget, MG-0103 from MethylGene, G2M-777 from TopoTarget and CG-781 from Celera, (gg) Anti colony-stimulating factor (GM-CSF) monoclonal antibodies such as KB-002 from KaloBios and (hh) Interferons comprising Interferon beta 1a such as Avonex from Biogen Idec, CinnoVex from CinnaGen and Rebif from EMD Serono, and Interferon beta 1 b such as Betaferon from Schering and Betaseron from Berlex.

When the salt of the invention is used for the treatment of rheumatoid arthritis, psoriatic arthritis, ankylosing spondilytis, multiple sclerosis, Wegener's granulomatosis, systemic lupus erythematosus, psoriasis and sarcoidosis it may be advantageous to use them in combination with other active compounds known to be useful in the treatment of such diseases such as rheumatoid arthritis, psoriatic arthritis, ankylosing spondilytis, multiple sclerosis, Wegener's granulomatosis, systemic lupus erythematosus, psoriasis and sarcoidosis.

Particularly preferred actives to be combined with the salt of the invention for treating or preventing rheumatoid arthritis, psoriatic arthritis, ankylosing spondilytis, multiple sclerosis, Wegener's granulomatosis, systemic lupus erythematosus, psoriasis or sarcoidosis are (a) Anti-TNF-alpha monoclonal antibodies such as Infliximab, Certolizumab pegol, Golimumab, Adalimumab and AME-527 from Applied Molecular Evolution, (b) TNF-alpha Antagonists such as Etanercept, Lenercept, Onercept and Pegsunercept, (c) Calcineurin (PP-2B) Inhibitors / INS Expression Inhibitors such as cyclosporine A, Tacrolimus and ISA-247 from Isotechnika, (d) IL-1 Receptor Antagonists such as Anakinra and AMG-719 from Amgen, (e) Anti-CD20 monoclonal antibodies such as Rituximab, Ofatumumab, Ocrelizumab and TRU-015 from Trubion Pharmaceuticals, (f) p38 Inhibitors such as AMG-548 (from Amgen), ARRY-797 (from Array Biopharma), Chlormethiazole edisylate, Doramapimod, PS-540446 (from BMS), SB-203580, SB-242235, SB-235699, SB-281832, SB-681323, SB-856553 (all from GlaxoSmithKline), KC-706 (from Kemia), LEO-1606, LEO-15520 (all from Leo), SC-80036, SD-06 (all from Pfizer), RWJ-67657 (from R.W. Johnson), RO-3201195, RO-4402257 (all from Roche), AVE-9940 (from Aventis), SCIO-323, SCIO-469 (all from Scios), TA-5493 (from Tanabe Seiyaku), and VX-745, VX-702 (all from Vertex) and the compounds claimed or described in Spanish patent applications numbers ES2303758 and ES2301380, (g) NF-kappaB (NFKB) Activation Inhibitors such as Sulfasalazine and Iguratimod, (h) Dihydrofolate Reductase (DHFR) Inhibitors such as Methrotexate, Aminopterin and CH-1504 from Chelsea and (hh) Interferons comprising Interferon beta 1a such as Avonex from Biogen Idec, CinnoVex from CinnaGen and Rebif from EMD Serono, and Interferon beta 1 b such as Betaferon from Schering and Betaseron from Berlex.

The combinations of the invention may be used in the treatment of disorders which are susceptible to amelioration by inhibition of the dihydroorotate dehydrogenase. Thus, the present application encompasses methods of treatment of these disorders, as well as the use of the combinations of the invention in the manufacture of a medicament for the treatment of these disorders.

Preferred examples of such disorders are rheumatoid arthritis, psoriatic arthritis, ankylosing spondilytis, multiple sclerosis, Wegener's granulomatosis, systemic lupus erythematosus, psoriasis and sarcoidosis, more preferably rheumatoid arthritis, psoriatic arthritis and psoriasis and most preferably rheumatoid arthritis.

The active compounds in the combinations of the invention may be administered by any suitable route, depending on the nature of the disorder to be treated, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The active compounds in the combination, i.e. the salts of the invention, and the other optional active compounds may be administered together in the same pharmaceutical composition or in different compositions intended for separate, simultaneous, concomitant or sequential administration by the same or a different route.

One execution of the present invention consists of a kit of parts comprising a salt of the invention together with instructions for simultaneous, concurrent, separate or sequential use in combination with another active compound useful in the treatment of rheumatoid arthritis, psoriatic arthritis, ankylosing spondilytis, multiple sclerosis, Wegener's granulomatosis, systemic lupus erythematosus, psoriasis and sarcoidosis.

Another execution of the present invention consists of a package comprising a salt of the invention and another active compound useful in the treatment of rheumatoid arthritis, psoriatic arthritis, ankylosing spondilytis, multiple sclerosis, Wegener's granulomatosis, systemic lupus erythematosus, psoriasis and sarcoidosis.

The pharmaceutical formulations may conveniently be presented in unit dosage form and may be prepared by any of the methods well known in the art of pharmacy.

Formulations of the present invention suitable for oral administration may be presented as discrete units such as capsules, sachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 2 µg and 150 µg of each therapeutically active ingredient. Alternatively, the active ingredient (s) may be presented without excipients.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

The amount of each active which is required to achieve a therapeutic effect will, of course, vary with the particular active, the route of administration, the subject under treatment, and the particular disorder or disease being treated.

Effective doses are normally in the range of 2-2000 mg of active ingredient per day. Daily dosage may be administered in one or more treatments, preferably from 1 to 4 treatments, per day. Preferably, the active ingredients are administered once or twice a day.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-α-day dosing occurred, or separately. Preferably at least two, and more preferably all, of the actives would be taken together at the same time. Preferably, at least two, and more preferably all actives would be administered as an admixture.

The following preparations forms are cited as composition (formulation) examples:

### COMPOSITION EXAMPLE 1

50,000 capsules, each containing 100 mg 2-[(3,5-difluoro-3'-methoxy-1,1'-biphenyl-4-yl)amino]nicotinic acid, tromethamine salt (active ingredient), were prepared according to the following formulation:

| | |
|---|---|
| Active ingredient | 5 Kg |
| Lactose monohydrate | 10 Kg |
| Colloidal silicon dioxide | 0.1 Kg |
| Corn starch | 1 Kg |
| Magnesium stearate | 0.2 Kg |

### Procedure

The above ingredients were sieved through a 60 mesh sieve, and were loaded into a suitable mixer and filled into 50,000 gelatine capsules.

### COMPOSITION EXAMPLE 2

50,000 tablets, each containing 50 mg of 2-[(3,5-difluoro-3'-methoxy-1,1'-biphenyl-4-yl)amino]nicotinic acid, tromethamine salt (active ingredient), were prepared from the following formulation:

| | |
|---|---|
| Active ingredient | 2.5 Kg |
| Microcrystalline cellulose | 1.95 Kg |
| Spray dried lactose | 9.95 Kg |
| Carboxymethyl starch | 0.4 Kg |
| Sodium stearyl fumarate | 0.1 Kg |
| Colloidal silicon dioxide | 0.1 Kg |

### Procedure

All the powders were passed through a screen with an aperture of 0.6 mm, then mixed in a suitable mixer for 20 minutes and compressed into 300 mg tablets using 9 mm disc and flat bevelled punches. The disintegration time of the tablets was about 3 minutes.

## Claims

1. A pharmaceutically acceptable crystalline addition salt of (i) an amine containing one or more hydroxyl and/or carboxylic groups with (ii) an amino nicotinic acid derivative of formula (I) 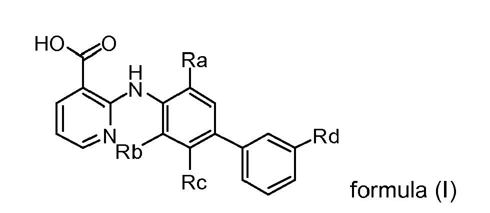 wherein
R^{a}, R^{b}, R^{c} and R^{d} independently represent groups selected from hydrogen atoms, halogen atoms, C₁₋₄ alkyl groups which may be optionally substituted by 1, 2 or 3 substituents selected from halogen atoms and hydroxy groups, and C₁₋₄ alkoxy groups which may be optionally substituted by 1, 2 or 3 substituents selected from halogen atoms and hydroxy groups,
and pharmaceutically acceptable solvates thereof.

2. A salt according to claim 1, wherein in the amino nicotinic acid derivative of formula (I) R^{a} represents a group selected from halogen atoms and C₁₋₄ alkoxy groups which may be optionally substituted by 1, 2 or 3 substituents selected from halogen atoms and hydroxy groups.

3. A salt according to claim 1 or claim 2, wherein in the amino nicotinic acid derivative of formula (I) R^{b} represents a group selected from hydrogen atoms and halogen atoms.

4. A salt according to any one of the preceding claims, wherein in the amino nicotinic acid derivative of formula (I) R^{c} represents a group selected from hydrogen atoms and C₁₋₄ alkyl groups which may be optionally substituted by 1, 2 or 3 substituents selected from halogen atoms and hydroxy groups.

5. A salt according to any one of the preceding claims, wherein in the amino nicotinic acid derivative of formula (I) R^{d} represents a group selected from hydrogen atoms and C₁₋₄ alkoxy groups which may be optionally substituted by 1, 2 or 3 substituents selected from halogen atoms and hydroxy groups.

6. A salt according to claim 1, wherein in the amino nicotinic acid derivative of formula (I) R^{a} represents a group selected from halogen atoms and C₁₋₄ alkoxy groups which may be optionally substituted by 1, 2 or 3 substituents selected from halogen atoms and hydroxy groups; R^{b} represents a group selected from hydrogen atoms and halogen atoms, R^{c} represents a group selected from hydrogen atoms and C₁₋₄ alkyl groups which may be optionally substituted by 1, 2 or 3 substituents selected from halogen atoms and hydroxy groups; and R^{d} represents a group selected from hydrogen atoms and C₁₋₄ alkoxy groups which may be optionally substituted by 1, 2 or 3 substituents selected from halogen atoms and hydroxy groups.

7. A salt according to any one of the preceding claims, wherein the amine containing one or more hydroxyl and/or carboxylic groups is selected from the group consisting of L-arginine, deanol, diethanolamine, diethylethanolamine, ethanolamine, meglumine, 2-morpholine ethanol, 1-(2-hydroxyethyl)-pyrrolidine, triethanolamine and tromethamine.

8. A salt according to claim 1, wherein the amine only contains one or more hydroxyl groups.

9. A salt according to claim 8, wherein the amine is selected from the group consisting of deanol, diethanolamine, diethylethanolamine, ethanolamine, meglumine, 2-morpholine ethanol, 1-(2-hydroxyethyl)-pyrrolidine, triethanolamine and tromethamine.

10. A salt according to claim 9, wherein the amine is selected from the group consisting of meglumine and tromethamine.

11. A salt according to claim 1, wherein the amine only contains one or more carboxylic groups.

12. A salt according to claim 11, wherein the amine is L-arginine.

13. A salt according to claim 1, wherein the amine containing one or more hydroxyl and/or carboxylic groups is selected from the group consisting of meglumine and tromethamine and in the amino nicotinic acid derivative of formula (I) R^{a} represents a group selected from halogen atoms and C₁₋₂ alkoxy groups which may be optionally substituted by 1, 2 or 3 halogen atoms; R^{b} represents a group selected from hydrogen atoms and halogen atoms, R^{c} represents a group selected from hydrogen atoms and C₁₋₂ alkyl groups which may be optionally substituted by 1, 2 or 3 substituents selected from halogen atoms and hydroxy groups; and R^{d} represents a group selected from hydrogen atoms and C₁₋₂ alkoxy groups which may be optionally substituted by 1, 2 or 3 substituents selected from halogen atoms and hydroxy groups.

14. A salt according to claim 1, wherein the amine containing one or more hydroxyl and/or carboxylic groups is L-arginine and in the amino nicotinic acid derivative of formula (I) R^{a} represents a group selected from halogen atoms and C₁₋₂ alkoxy groups which may be optionally substituted by 1, 2 or 3 halogen atoms; R^{b} represents a group selected from hydrogen atoms and halogen atoms, R^{c} represents a group selected from hydrogen atoms and C₁₋₂ alkyl groups which may be optionally substituted by 1, 2 or 3 substituents selected from halogen atoms and hydroxy groups; and R^{d} represents a group selected from hydrogen atoms and C₁₋₂ alkoxy groups which may be optionally substituted by 1, 2 or 3 substituents selected from halogen atoms and hydroxy groups.

15. A salt according to claim 1 selected from the group consisting of:
2-{[3'-ethoxy-3-(trifluoromethoxy)-1,1'-biphenyl-4-yl]amino}nicotinic acid, meglumine salt,
2-[(3,5-difluoro-2-methyl-1,1'-biphenyl-4-yl)amino]nicotinic acid, meglumine salt,
2-[(3,5-difluoro-2-methyl-1,1'-biphenyl-4-yl)amino]nicotinic acid, tromethamine salt,
2-[(3,5-difluoro-3'-methoxy-1,1'-biphenyl-4-yl)amino]nicotinic acid, meglumine salt,
2-[(3,5-difluoro-3'-methoxy-1,1'-biphenyl-4-yl)amino]nicotinic acid, tromethamine salt,
2-[(3,5-difluoro-3'-methoxy-1,1'-biphenyl-4-yl)amino]nicotinic acid, L-arginine salt,
and pharmaceutically acceptable solvates thereof.

16. A pharmaceutical composition comprising a therapeutically effective amount of a salt as defined in any one of the preceding claims and a pharmaceutically acceptable carrier.

17. A pharmaceutical composition according to claim 16, wherein the composition further comprises a therapeutically effective amount of one or more other therapeutic agents.

18. The pharmaceutical composition of claim 17, wherein the other therapeutic agent is selected from:
a) Anti-TNF-alpha monoclonal antibodies such as Infliximab, Certolizumab pegol, Golimumab, Adalimumab and AME-527 from Applied Molecular Evolution
b) TNF-alpha Antagonists such as Etanercept, Lenercept, Onercept and Pegsunercept
c) Calcineurin (PP-2B) Inhibitors / INS Expression Inhibitors such as cyclosporine A, Tacrolimus and ISA-247 from Isotechnika
d) IL-1 Receptor Antagonists such as Anakinra and AMG-719 from Amgen
e) Anti-CD20 monoclonal antibodies such as Rituximab, Ofatumumab, Ocrelizumab and TRU-015 from Trubion Pharmaceuticals
f) p38 Inhibitors such as AMG-548 (from Amgen), ARRY-797 (from Array Biopharma), Chlormethiazole edisylate, Doramapimod, PS-540446 (from BMS), SB-203580, SB-242235, SB-235699, SB-281832, SB-681323, SB-856553 (all from GlaxoSmithKline), KC-706 (from Kemia), LEO-1606, LEO-15520 (all from Leo), SC-80036, SD-06 (all from Pfizer), RWJ-67657 (from R.W. Johnson), RO-3201195, RO-4402257 (all from Roche), AVE-9940 (from Aventis), SCIO-323, SCIO-469 (all from Scios), TA-5493 (from Tanabe Seiyaku), and VX-745 and VX-702 (all from Vertex)
g) NF-kappaB (NFKB) Activation Inhibitors such as Sulfasalazine and Iguratimod
h) Dihydrofolate Reductase (DHFR) Inhibitors such as Methrotexate, Aminopterin and CH-1504 from Chelsea
hh) Interferons comprising Interferon beta 1 a such as Avonex from Biogen Idec, CinnoVex from CinnaGen and Rebif from EMD Serono, and Interferon beta 1 b such as Betaferon from Schering and Betaseron from Berlex.

19. A combination comprising a salt as defined in any one of claims 1 to 15 and one or more other therapeutic agents, as defined in claim 18.

20. A salt according to any one of claims 1 to 15, a pharmaceutical composition according to any one of claims 16 to 18 or a combination according to claim 19 for use in the treatment of a pathological condition or disease susceptible to amelioration by inhibition of dihydroorotate dehydrogenase.

21. A salt, a pharmaceutical composition or a combination according to claim 20 wherein the pathological condition or disease is selected from rheumatoid arthritis, psoriatic arthritis, ankylosing spondilytis, multiple sclerosis, Wegener's granulomatosis, systemic lupus erythematosus, psoriasis and sarcoidosis.

22. Use of a salt according to any one of claims 1 to 15, a pharmaceutical composition according to any one of claims 16 to 18 or a combination according to claim 19 for the manufacture of a medicament for the treatment of a pathological condition or disease as defined in claims 20 or 21.

23. A method of treatment of a subject afflicted with a pathological condition or disease as defined in claim 20 or 21, which comprises administering to said subject an effective amount of a salt according to any one of claims 1 to 15, a pharmaceutical composition according to any one of claims 16 to 18 or a combination according to claim 19.
